# EUROPÄISCHE PATENTSCHRIFT

(11) **EP 0 767 830 B1**
(45) Veröffentlichungstag und Bekanntmachung des Hinweises auf die Patenterteilung: **18.11.1998**
(21) Anmeldenummer: 95924278.5
(22) Anmeldetag: 19.06.1995
(51) Int. Cl.: C11D 3/386, C12N 9/98

(54) **HERSTELLUNG EINES MEHRENZYMGRANULATS**
PRODUCTION OF MULTIPLE ENZYME GRANULATES
PRODUCTION DE GRANULES D'ENZYMES MULTIPLES

(30) Priorität: 28.06.1994 DE 4422433
(43) Veröffentlichungstag der Anmeldung: 16.04.1997
(73) Patentinhaber: Henkel Kommanditgesellschaft auf Aktien, 40191 Düsseldorf (DE)
(72) Erfinder: MEINE, Georg, D-40822 Mettmann (DE); MAURER, Karl-Heinz, D-40699 Erkrath (DE); WEISS, Albrecht, D-40764 Langenfeld (DE); PAATZ, Kathleen, D-40589 Düsseldorf (DE); HAAS, Ulrich, D-40699 Erkrath (DE); BÖCKER, Monika, D-42799 Leichlingen (DE)
(86) Internationale Anmeldenummer: EP9502363
(87) Internationale Veröffentlichungsnummer: WO9600773

(56) Entgegenhaltungen:
- DE-A- 2 030 531
- DE-A- 2 137 042
- DE-A- 4 041 752
- US-A- 3 737 376

## Beschreibung

Die Erfindung betrifft ein Enzymgranulat, das mindestens zwei verschiedene Enzyme enthält, ein Verfahren zu seiner Herstellung und die Verwendung des Granulats in festen oder flüssigen Wasch- und Reinigungsmitteln.

Enzyme, insbesondere Proteasen, finden ausgedehnte Verwendung in Wasch-, Waschhilfs-und Reinigungsmitteln. Üblicherweise kommen die Enzyme dabei nicht als Reinsubstanzen, sondern in Mischungen mit einem Verdünnungs- und Trägermaterial zum Einsatz. Mischt man solche Enzymzubereitungen üblichen Waschmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation gemäß der deutschen Offenlegungsschrift DT 16 17 190 beziehungsweise durch Aufkleben mit nichtionischen Tensiden gemäß der deutschen Offenlegungsschrift DT 16 17 188 oder wäßrigen Lösungen von Celluloseethern gemäß der deutschen Offenlegungschrift DT 17 87 568 führt nicht zu einer nennenswerten Verbesserung der Lagerstabilität, da sich die empfindlichen Enzyme in solchen Aufmischungen in der Regel auf der Oberfläche der Trägersubstanz befinden. Zwar kann die Lagerstabilität der Enzyme wesentlich erhöht werden, wenn man die Enzyme mit dem Trägermaterial umhüllt beziehungsweise in dieses einbettet und anschließend durch Extrudieren, Pressen und Spheronisieren in die gewünschte Partikelform überführt, wie zum Beispiel in der deutschen Patentschrift DE 16 17 232, der deutschen Offenlegungsschrift DT2032768 und den deutschen Auslegeschriften DE 21 37 042 und DE 21 37 043 beschrieben. Derartige Enzymzubereitungen besitzen jedoch nur mangelhafte Löslichkeitseigenschaften. Die ungelösten Partikel können sich im Waschgut verfangen und dieses verunreinigen beziehungsweise sie werden ungenutzt in das Abwasser überführt. Aus der deutschen Offenlegungsschrift DT 18 03 099 bekannte Einbettungsmittel, die aus einem Gemisch fester Säuren beziehungsweise saurer Salze und Carbonaten beziehungsweise Bicarbonaten bestehen und bei Wasserzusatz zerfallen, verbessern zwar das Lösungsvermögen, sind aber ihrerseits sehr empfindlich gegen Feuchtigkeit und erfordern daher zusätzliche Schutzmaßnahmen. Ein weiterer Nachteil der vorgenannten Zubereitung ist darin zu sehen, daß die Enzyme nur in Form trockener Pulver verarbeitet werden können. Die üblicherweise bei der Enzymherstellung anfallenden Fermentbrühen lassen sich in dieser Form nicht einsetzen, sondern müssen zuvor entwässert werden.

Aus der europäischen Patentschrift EP 168 526 sind Enzymgranulate bekannt, die in Wasser quellfähige Stärke, Zeolith und wasserlösliches Granulierhilfsmittel enthalten. In diesem Dokument wird ein das obengenannte Problem umgehendes Herstellungsverfahren für derartige Formulierungen vorgeschlagen, das im wesentlichen darin besteht, eine von unlöslichen Bestandteilen befreite Fermenterlösung aufzukonzentrieren, mit den genannten Zuschlagstoffen zu versetzten und das entstandene Gemisch zu granulieren. Das Verfahren mit dem dort vorgeschlagenen Zuschlagstoffgemisch wird vorteilhaft mit Fermentationslösungen durchgeführt, die auf einen relativ hohen Trockensubstanzgehalt, beispielsweise 55 Gew.-%, aufkonzentriert worden sind.

Aus der internationalen Patentanmeldung WO 92/11347 sind Enzymgranulate zum Einsatz in körnigen Wasch- und Reinigungsmitteln bekannt, die 2 Gew.-% bis 20 Gew.-% Enzym, 10 Gew.-% bis 50 Gew.-% quellfähige Stärke, 5 Gew.-% bis 50 Gew.-% wasserlösliches organisches Polymer als Granulierhilfsmittel, 10 Gew.-% bis 35 Gew.-% Getreidemehl und 3 Gew.-% bis 12 Gew.-% Wasser enthalten. Durch derartige Zuschlagstoffe wird die Enzymverarbeitung ohne größere Aktivitätsverluste möglich und auch die Lagerbeständigkeit der Enzyme in den Granulaten ist zufriedenstellend.

Wie anhand der genannten Dokumente beispielhaft geschildert, existiert ein breiter Stand der Technik auf dem Gebiet der Herstellung von granularen Enzymzubereitungen, so daß dem Fachmann verschiedene Möglichkeiten zur teilchenförmigen Konfektionierung von einzelnen Enzymen zur Verfügung stehen. Die genannten Methoden versagen jedoch, wenn es darum geht, zwei oder mehrere Enzyme, welche miteinander reagieren können, in dasselbe Granulat einzuarbeiten. Insbesondere stellt sich dieses Problem im Zusammenhang mit Protease, welche als proteinabbauendes Enzym naturgemäß in der Lage ist, ein gleichzeitig vorhandenes zweites und/oder weiteres Enzym zu zersetzen. Wenn diese Zersetzung bei der Herstellung und/oder während der Lagerung der Enzymgranulate eintritt, ist die Wirkung des zweiten und/oder weiteren Enzyms unter Anwendungsbedingungen nicht mehr gewährleistet.

Auch zur Lösung dieses Problems gibt es Ansätze im Stand der Technik. So wird in der internationalen Patentanmeldung WO 90/09440 ein Zweienzymgranulat dadurch hergestellt, daß man einen protease- und cellulosehaltigen Kern mit insgesamt 10 Schichten (abwechselnd Stearinsäure-/Palmitinsäureglycerid und Kaolin) überzieht, wobei in den Beispielen die Menge des Schutzüberzugsmaterials diejenige des Kerns übersteigt, anschließend ein Gemisch aus einem zweiten Enzym, einem Bindemittel, einem Füllmaterial und einem Granulierhilfsmittel aufbringt und schließlich mit einer äußeren Coatingschicht umhüllt. Ein derartiges Herstellverfahren ist wegen des hohen Bedarfs an Trennmaterial zwischen dem enzymhaltigen Kern und der das zweite Enzym enthaltenden, weiter außen liegenden Schicht ungünstig. Von Nachteil könnte auch sein, daß unter Anwendungsbedingungen sich zuerst das außen liegende Enzym in Lösung begibt, und erst später das zweite Enzym aus dem Kem freigesetzt wird, so daß nicht beide Enzym gleichzeitig ihre Wirkung entfalten können.

Die US-amerikanische Patentschrift US-A-3 737376 offenbart ein freifließendes Enzymgranulat, das Protease und Amylase enthalten kann und durch inniges Vermischen der offensichtlich in Pulverform vorliegenden Einzelenzyme mit Petrolatum als einer Staub und Feuchtigkeit inhibierenden Substanz hergestellt wird.

Aus der nicht vorveröffentlichten deutschen Patentanmeldung DE43 29 463 ist ein Herstellverfahren für Mehrenzymgranulate bekannt, bei dem zwei separat hergestellte, jeweils ein Enzym enthaltende Granulate mit unterschiedlichen Korngrößen in einem anschließenden Cogranulationsschritt agglomeriert werden.

Aus der europäischen Patentanmeldung EP 304 332 ist bekannt, daß man ein enzymhaltiges Basisgranulat mit pulverförmigen Komponenten, welche ein zweites Enzym umfassen, umhüllen kann. Diese Art der Herstellung von Mehrenzymgranulaten führt jedoch oft zu mangelnder Stabilität des zweiten, in der äußeren Schicht befindlichen Enzyms, das überdies nachteiligerweise zuvor in feinteiliger Pulverform vorliegend zubereitet werden muß. Auch bei dieser Variante liegen in der Regel nicht beide Enzyme gleichzeitig freigesetzt im Wasch- beziehungsweise Reinigungsmedium vor.

Es bestand daher die Aufgabe, ein möglichst einfaches Herstellungsverfahren für teilchenförmige Enzymzubereitungen zu entwickeln, welche mindestens zwei verschiedene, miteinander reagierende Enzyme enthalten, wobei die Enzyme ohne Aktivitätsverlust in das Mehrenzymgranulat eingearbeitet werden müssen und dort lagerstabil verbleiben sollen. Dies gelang überraschenderweise im wesentlichen durch den Einsatz eines kompetitiv reversibel gehemmten Enzyms in wäßriger Formulierung, die mit weiterem Enzym sowie Zuschlagstoffen vermischt und extrudiert wird.

Gegenstand der Erfindung ist demgemäß ein Verfahren zur Herstellung von Enzymgranulaten, die mindestens zwei verschiedene Enzyme enthalten, durch Vermischen einer wäßrigen, ein erstes Enzym enthaltenden Flüssigkeit, die gegegebenfalls eine von unlöslichen Bestandteilen befreite und aufkonzentrierte Fermentationsbrühe sein kann, mit einem kompetitiven Inhibitor für dieses Enzym, anschließendes Vermischen des Primärenzyms mit dem zweiten Enzym beziehungsweise weiteren Enzymen, Zumischen von organischem und/oder anorganischem Trägermaterial, Extrusion der so erhaltenen Mischung aus Enzymen und Zuschlagstoffen durch eine Lochplatte mit anschließendem Schneidegerät, gegebenenfalls Sphäronisierung des Extrudats in einem Rondiergerät sowie Trocknung, und gegebenenfalls Aufbringen eines gewünschtenfalls Farbstoff und/oder Pigment enthaltenden Überzugs.

Ein weiterer Gegenstand der Erfindung ist die Verwendung der so erhältlichen Mehrenzymgranulate in insbesondere teilchenförmigen Wasch- oder Reinigungsmitteln.

Durch das erfindungsgemäße Verfahren erhält man ein für die Einarbeitung in Wasch- und Reinigungsmittel geeignetes Enzymgranulat, welches dadurch gekennzeichnet ist, daß es mindestens zwei verschiedene, insbesondere miteinander zu reagieren vermögende, das heißt nicht miteinander verträgliche Enzyme in homogener Verteilung enthält. Die nicht miteinander verträglichen Enzyme können durch das erfindungsgemäße Verfahren gemeinsam in ein Granulat eingearbeitet werden, in dem sie weitgehend homogen vorliegen und dennoch sich gegenseitig nicht negativ beeinflussen. Wesentlich hierfür ist, daß das Sekundärenzym nicht direkt der konzentrierten wäßrigen Primärenzymlösung zugegeben wird, sondern daß das Primärenzym in dem wäßrigen Konzentrat zuerst durch einen kompetitiven Inhibitor reversibel inaktiviert wird. Unter Anwendungsbedingungen, das heißt in wäßrigen Waschbeziehungsweise Reinigungslaugen, wird die Inhibierung des Primärenzyms durch das Auflösen der Granulatstruktur und die Ab- beziehungsweise Auflösung des Inhibitors aufgehoben und Primär- sowie Sekundärenzym entfalten mehr oder weniger gleichzeitig ihre Wirkung. Vorzugsweise handelt es sich bei dem Primärenzym um Protease und bei dem Sekundärenzym um Amylase, Lipase, Cellulase, Hemicellulase, Oxidase, Peroxidase oder um Mischungen aus diesen. Das Sekundärenzym kann in flüssiger Form, beispielsweise als handelsübliches Konzentrat, oder in fester, konfektionierter Form, beispielsweise als handelsübliches Granulat, dem Primärenzym zugemischt werden.

Als im erfindungsgemäß hergestellten Enzymgranulat enthaltenes Primärenzym kommt in erster Linie aus Mikroorganismen, wie Bakterien oder Pilzen, gewonnene Protease in Frage. Sie kann in bekannter Weise durch Fermentationsprozesse aus geeigneten Mikroorganismen gewonnen werden, die zum Beispiel in den deutschen Offenlegungsschriften DE 19 40 488, DE 20 44 161, DE 22 01 803 und DE 21 21 397, den US-amerikanischen Patentschriften US 3 632 957 und US 4 264 738, der europäischen Patentanmeldung EP 006 638 sowie der internationalen Patentanmeldung WO 91/02792 beschrieben sind. Proteasen sind im Handel beispielsweise unter den Namen BLAP^{(R)}, Savinase^{(R)}, Esperase^{(R)}, Maxatase^{(R)}, Optimase^{(R)}, Alcalase^{(R)}, Durazym^{(R)} oder Maxapem^{(R)} erhältlich.

Das Primärenzym ist in den erfindungsgemäßen Extrudaten vorzugsweise in Mengen von 1 Gew.-% bis 6 Gew.-% enthalten. Falls es sich bei dem erfindungsgemäßen Enzymgranulat um eine proteasehaltige Formulierung handelt, beträgt die Proteaseaktivität vorzugsweise 50 000 Proteaseeinheiten (PE, bestimmt nach der in Tenside 7 (1970), 125 beschriebenen Methode) bis 350 000 PE, insbesondere 100 000 PE bis 250 000 PE, pro Gramm Enzymgranulat.

Die als Sekundärenzym beziehungsweise als Sekundärenzymkomponente im erfindungsgemäßen Verfahren einsetzbare Lipase kann aus Humicola lanuginosa, wie beispielsweise in den europäischen Patentanmeldungen EP 258 068, EP 305 216 und EP 341 947 beschrieben, aus Bacillus-Arten, wie beispielsweise in der internationalen Patentanmeldung WO 91/16422 oder der europäischen Patentanmeldung EP 384 717 beschrieben, aus Pseudomonas-Arten, wie beispielsweise in den europäischen Patentanmeldungen EP 468 102, EP 385 401, EP 375 102, EP 334 462, EP 331 376, EP 330 641, EP 214 761, EP 218 272 oder EP 204 284 oder der internationalen Patentanmeldung WO 90/10695 beschrieben, aus Fusarium-Arten, wie beispielsweise in der europäischen Patentanmeldung EP 130 064 beschrieben, aus Rhizopus-Arten, wie beispielsweise in der europäischen Patentanmeldung EP 117 553 beschrieben, oder aus Aspergillus-Arten, wie beispielsweise in der europäischen Patentanmeldung EP 167 309 beschrieben, gewonnen werden. Geeignete Lipasen sind beispielsweise unter den Namen Lipolase^{(R)}, Lipozym^{(R)}, Lipomax^{(R)}, Amano^{(R)}-Lipase, Toyo-Jozo^{(R)}-Lipase, Meito^{(R)}-Lipase und Diosynth^{(R)}-Lipase im Handel erhältlich. Lipase wird im erfindungsgemäßen Verfahren vorzugsweise in solchen Mengen eingesetzt, daß das Mehrenzymgranulat 1 KLU/g ("Kilo-Lipase-Units" pro Gramm gemäß der Standard-Methode der Firma Novo, basierend auf der enzymatischen Hydrolyse von Tributyrin, wie in der Novo Nordisk Publikation AF 95 beschrieben) bis 80 KLU/g, insbesondere 1,5 KLU/g bis 60 KLU/g und besonders bevorzugt 2 KLU/g bis 30 KLU/g aufweist.

Besonders für den Einsatz in Geschirrspülmitteln, insbesondere zum maschinellen Einsatz, geeignet ist ein Mehrenzymgranulat, welches als Primärenzym Protease und als Sekundärenzym Amylase enthält. Geeignete Amylasen sind beispielsweise unter den Namen Maxamyl^{(R)} und Termamyl^{(R)} handelsüblich. Amylase wird im erfindungsgemäßen Verfahren vorzugsweise in solchen Mengen eingesetzt, daß das Mehrenzymgranulat 1 KNU/g ("Kilo-Novo-Units" pro Gramm gemäß der Standard-Methode der Firma Novo, wobei 1 KNU die Enzymmenge ist, die 5,26 g Stärke bei pH 5,6 und 37 °C abbaut, basierend auf der von P. Bernfeld in S.P. Colowick und N.D. Kaplan, Methods in Enzymology, Band 1, 1955, Seite 149 beschriebenen Methode) bis 100 KNU/g, insbesondere 2 KNU/g bis 60 KNU/g und besonders bevorzugt 5 KNU/g bis 50 KNU/g aufweist.

Die als Sekundärenzym beziehungsweise als Sekundärenzymkomponente einsetzbare Cellulase kann ein aus Bakterien oder Pilzen gewinnbares Enzym sein, welches ein pH-Optimum vorzugsweise im schwach sauren bis schwach alkalischen Bereich von 6 bis 9,5 aufweist. Derartige Cellulasen sind beispielsweise aus den deutschen Offenlegungsschriften DE 31 17 250, DE 32 07 825, DE 32 07 847, DE 33 22 950 oder den europäischen Patentanmeldungen EP 265 832, EP 269 977, EP 270 974, EP 273 125 sowie EP 339550 bekannt. Sie werden vorzugsweise in solchen Mengen eingesetzt, daß das fertige Mehrenzymgranulat eine cellulolytische Aktivität von 50 CEVU/g ("Cellulose Viscosity Units" pro Gramm, basierend auf der enzymatischen Hydrolyse von Carboxymethylcellulose bei pH 9,0 und 40 □C, wie in der Novo Nordisk Publikation AF 253 beschrieben) bis 1250 CEVU/g und insbesondere 100 CEVU/g bis 1000 CEVU/g aufweist.

Das erfindungsgemäße Herstellungsverfahren beinhaltet das Vermischen eines in flüssiger Form, beispielsweise einer wäßrigen, gegebenenfalls von unlöslichen Bestandteilen befreiten Fermentationsbrühe, vorliegenden ersten Enzyms, das vorzugsweise einen Wassergehalt unter 35 Gew.-%, insbesondere von 5 Gew.-% bis 30Gew.-% aufweist, mit kompetitivem Inhibitor für dieses Enzym. Zu derartigen Inhibitoren gehören mehrwertige Alkohole, insbesondere Glycerin, Propylenglykol, Aminoalkohole, beispielsweise Mono-, Di-, Triethanol- und -propanolamin und deren Mischungen, niedere Carbonsäuren, wie beispielsweise aus den europäischen Patentanmeldungen EP 376 705 und EP 378 261 bekannt, Borsäure beziehungsweise Alkaliborate, Borsäure-Carbonsäure-Kombinationen, wie beispielsweise aus der europäischen Patentanmeldung EP 451 924 bekannt, Borsäureester, wie beispielsweise aus der internationalen Patentanmeldung WO 93/11215 oder der europäischen Patentanmeldung EP 511 456 bekannt, Boronsäurederivate, wie beispielsweise aus der europäischen Patentanmeldung EP 583 536 bekannt, Calciumsalze, beispielsweise die aus der europäischen Patentschrift EP28865 bekannte Ca-Ameisensäure-Kombination, Magnesiumsalze, wie beispielsweise aus der europäischen Patentanmeldung EP 378 262 bekannt, und/oder schwefelhaltige Reduktionsmittel, wie beispielsweise aus den europäischen Patentanmeldungen EP080748 oder EP080223 bekannt. Die genannten Substanzen werden vorzugsweise in Mengen von 20 Gew.-% bis 60 Gew.-%, insbesondere von 35 Gew.-% bis 50Gew.-%, bezogen auf entstehendes Gemisch aus wäßrigem Enzym und Inhibitor, zugegeben.

Als Trägermaterialien für das Enzymgemisch, die direkt anschließend, insbesondere aber erst nach Zufügen des Sekundärenzyms zugemischt werden können, sind im Prinzip alle organischen oder anorganischen pulverförmigen Substanzen brauchbar, welche die Enzyme nicht oder nur tolerierbar wenig zerstören beziehungsweise nur reversibel desaktivieren und unter Extrusionsbedingungen stabil sind. Zu derartigen Substanzen gehören beispielsweise Cellulose, Maltodextrose, Saccharose, Invertzucker, Glukose, Stärken, Getreidemehle, Celluloseether, Alkalialumosilikat, insbesondere Zeolith, Schichtsilikat, zum Beispiel Bentonit oder Smectit, und wasserlösliche anorganische oder organische Salze, zum BeispielAlkalichlorid, Alkalisulfat, Alkalicarbonat oder Alkaliacetat, wobei Natrium oder Kalium die bevorzugten Alkalimetalle sind. Bevorzugt wird ein Trägermaterialgemisch aus Stärke, Getreidemehl, pulverförmiger Cellulose und Saccharose und gegebenenfalls Celluloseether sowie Alkalicarbonat eingesetzt. Falls das Sekundärenzym in fester Form eingesetzt wird, liegt es in der Regel als mit Hilfe derartiger Trägermaterialien konfektioniertes Pulver oder Granulat vor. In einer Ausführungsform des erfindungsgemäßen Verfahrens kann in diesem Fall auf die separate Zugabe von Trägermaterial verzichtet werden.

Bei der als Trägermaterial beziehungsweise Trägermaterialkomponente geeigneten Stärke handelt es sich vorzugsweise um Maisstärke, Reisstärke, Kartoffelstärke oder Gemische aus diesen, wobei der Einsatz von Maisstärke besonders bevorzugt ist. Stärke ist in dem Trägermaterial für das Enzymgemisch vorzugsweise in Mengen von 20 Gew.-% bis 80 Gew.-%, insbesondere von 25 Gew.-% bis 75Gew.-%, jeweils bezogen auf gesamtes Trägermaterial, enthalten. Dabei beträgt die Summe der Mengen der Stärke und des Mehls vorzugsweise nicht über 95 Gew.-%, insbesondere 60 Gew.-% bis 95 Gew.-%. Bei dem Getreidemehl handelt es sich insbesondere um ein aus Weizen, Roggen, Gerste oder Hafer herstellbares Produkt oder um ein Gemisch dieser Mehle, wobei Vollkornmehle bevorzugt sind. Unter einem Vollkornmehl wird im Rahmen der Erfindung ein nicht voll ausgemahlenes Mehl verstanden, das aus ganzen, ungeschälten Körnern hergestellt worden ist oder zumindest überwiegend aus einem derartigen Produkt besteht, wobei der Rest aus voll ausgemahlenem Mehl beziehungsweise Stärke besteht. Vorzugsweise werden handelsübliche Weizenmehl-Qualitäten, wie Type 450 oder Type 550, eingesetzt. Auch die Verwendung von Mehlprodukten der zu vorgenannten Stärken führenden Getreidearten ist möglich, wenn darauf geachtet wird, daß die Mehle aus den ganzen Körnern hergestellt worden sind. Durch die Mehlkomponente des Zuschlagstoffgemisches wird bekanntermaßen eine wesentliche Geruchsreduzierung der Enzymzubereitung erreicht, welche die Geruchsverminderung durch die Einarbeitung gleicher Mengen entsprechender Stärkearten bei weitem übertrifft. Derartiges Getreidemehl ist im Trägermaterial für das Primärenzym vorzugsweise in Mengen von 10 Gew.-% bis 35 Gew.-%, insbesondere von 15 Gew.-% bis 20 Gew.-% enthalten.

Als zusätzliche Bestandteile des Trägermaterials können Granulierhilfsmittel vorhanden sein, zu denen beispielsweise Cellulose- oder Stärkeether, wie Carboxymethylcellulose, Carboxymethylstärke, Methylcellulose, Hydroxyethylcellulose, Hydroxypropylcellulose sowie entsprechende Cellulosemischether, Gelatine, Casein, Traganth oder andere in Wasser lösliche beziehungsweise gut dispergierbare Oligomere oder Polymere natürlichen oder synthetischen Ursprungs verwendet werden. Zu den synthetischen wasserlöslichen Polymeren sind Alkyl- bzw. Alkenylpolyethoxylate, Polyethylenglykole, Polyacrylate, Polymethacrylate, Copolymere der Acrylsäure mit Maleinsäure oder vinylgruppenhaltigen Verbindungen, ferner Polyvinylalkohol, teilverseiftes Polyvinylacetat und Polyvinylpyrrolidon zu rechnen. Polyethylenglykole werden vorzugsweise unter denjenigen mit mittleren Molmassen von 200 bis 3000 ausgewählt. Soweit es sich bei den vorgenannten Granulierhilfsmitteln um solche mit Carboxylgruppen handelt, liegen diese normalerweise in Form ihrer Alkalisalze, insbesondere ihrer Natriumsalze vor. Derartige Granulierhilfsmittel können in den erfindungsgemäß geeigneten Enzym-Vorgemischen in Mengen bis zu 10 Gew.-%, insbesondere von 0,5 Gew.-% bis 8 Gew.-%, jeweils bezogen auf zu extrudierendes Mehrenzymgemisch, enthalten sein. Der Substitutionsgrad bei vorzugsweise eingesetzten Carboxymethylcellulosen liegt im Bereich von 0,8 bis 0,95, da bei deren Einsatz besonders feste Granulatkörner erhalten werden beziehungsweise geringere Mengen erforderlich sind, um eine bestimmte Granulatfestigkeit zu erreichen, als bei Einsatz von Celluloseethern mit niedrigerem Substitutionsgrad. Außerdem kann durch den Einsatz der genannten höhersubstituierten Carboxymethylcellulose bei der Herstellung der Granulate im Extrusionsschritt ein höherer Durchsatz durch den Extruder erreicht werden. Unter dem Substitutionsgrad der Carboxymethylcellulose ist die Zahl der veretherten, eine Carboxymethylgruppe tragenden Sauerstoffatome pro Saccharid-Monomer der Cellulose zu verstehen.

Das fest oder vorzugsweise in flüssiger Form vorliegende Sekundärenzym kann anschließend oder vor der Zugabe des Trägermaterials beziehungsweise Granulationshilfsmittels dem Primärenzym zugesetzt werden. Die neben dem zweiten Enzym gegebenenfalls zusätzlich enthaltenen weiteren Bestandteile des Sekundärenzyms sind nicht kritisch, obwohl für den bevorzugten Einsatz des erfindungsgemäßen Enzymgranulats in Wasch- und Reinigungsmitteln übliche Inhaltsstoffe solcher Mittel oder zumindest mit diesen verträgliche Stoffe bevorzugt sind. Falls das Sekundärenzym in fester Form, das heißt in Abmischung mit Trägern oder Verschnittmitteln zugegeben wird, enthält diese Zugabekomponente vorzugsweise anorganisches Salz, insbesondere Alkalisulfat und/oder -chlorid, in Mengen von, jeweils bezogen auf Sekundärenzym-Konfektionierungsform, 30 Gew.-% bis 80 Gew.-%, faser- oder pulverförmige Cellulose in Mengen von 2 Gew.-% bis 40 Gew.-%, und Bindemittel, insbesondere Dextrose, Saccharose, Polyvinylalkohol und/oder Polyvinylpyrrolidon, in Mengen von 0,1 Gew.-% bis 15 Gew.-%. Im erfindungsgemäßen Herstellungsverfahren können auch fertig konfektionierte Enzymgranulate, welche das Sekundärenzym enthalten, eingesetzt werden. Möglich ist demnach, das das zweite Enzym enthaltende Teilchen durch ein Extrusionsverfahren, wie beispielsweise in der internationalen Patentanmeldung WO 92/11347 oder der europäischen Patentschrift EP 168 526 beschrieben, herzustellen. Vorzugsweise stellt man teilchenförmig konfektionierte Sekundärenzyme durch Aufbaugranulation aus einem anorganischen und/oder organischen Trägermaterial und wäßriger Enzymlösung her. Ein solches Vorgehen unter Verwendung von anorganischem Salz und Cellulosefasern im Trägermaterial und Wasser und/oder wachsartiger Substanz als Bindemittel ist beispielsweise in der deutschen Patentschrift DE 27 30 481 beschrieben.

Zur Herstellung der erfindungsgemäßen Enzymgranulate geht man vorzugsweise von wäßrigen Primärenzym-Fermentbrühen aus, die von unlöslichen Begleitstoffen, beispielsweise durch Mikrofiltration, befreit werden. Die Mikrofiltration wird dabei vorzugsweise als Querstrom-Mikrofiltration unter Verwendung poröser Rohre mit Mikroporen größer 0,1 µm, Fließgeschwindigkeiten der Konzentratlösung von mehr als 2 m/s und einem Druckunterschied zur Permeatseite von unter 5 bar durchgeführt, wie zum Beispiel in der europäischen Patentanmeldung EP 200 032 beschrieben. Anschließend wird das Mikrofiltrationspermeat vorzugsweise durch Ultrafiltration, gegebenenfalls mit anschließender Vakuumeindampfung, aufkonzentriert. Die Aufkonzentration wird dabei vorzugsweise so geführt, daß man zu Wassergehalten nicht über 35 Gew.-% gelangt. Das Konzentrat wird mit dem Sekundärenzym und einem zweckmäßigerweise zuvor hergestellten trockenen, pulverförmigen bis körnigen Gemisch der oben beschriebenen Trägermaterialien beziehungsweise Extrusionshilfsmittel vermischt, wobei auch die umgekehrte Zumischreihenfolge oder das gleichzeitige Zusetzen möglich ist. Diese Zuschlagstoffe werden vorzugsweise so unter den genannten Trägermaterialien und Extrudierhilfsmitteln ausgewählt, daß das entstehende Mehrenzymextrudat ein Schüttgewicht von 700 g/l bis 1200 g/l aufweist. Der Wassergehalt der zu extrudierenden Mischung sollte so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt. Das Mehrenzymgemisch wird in im Prinzip bekannter Weise anschließend in einem Kneter sowie einem angeschlossenen Extruder zu einer plastischen Masse verarbeitet, wobei als Folge der mechanischen Bearbeitung sich die Masse auf Temperaturen zwischen 40°C und 60°C, insbesondere 45°C bis 55°C erwärmen kann. Das den Extruder verlassende Gut wird durch eine Lochscheibe mit nachfolgendem Abschlagmesser geführt und dadurch zu zylinderförmigen Partikeln definierter Größe zerkleinert. Zweckmäßigerweise beträgt der Durchmesser der Bohrungen in der Lochscheibe 0,7 mm bis 1,2 mm, vorzugsweise 0,8 mm bis 1,0 mm. Das Verhältnis zwischen Länge und Dicke des Extrudats liegt vorzugsweise im Bereich von 0,9 bis 1,1, insbesondere bei 1,0. Die in dieser Form vorliegenden Partikel können, gegebenenfalls nach einem Trocknungsschritt, direkt in Wasch- und Reinigungsmittel eingearbeitet werden. Es hat sich jedoch als vorteilhaft erwiesen, die den Extruder und Zerhacker verlassenden zylindrischen Partikel anschließend zu sphäronisieren, das heißt sie in geeigneten Vorrichtungen abzurunden und zu entgraten. Ein solches Sphäronisierungsverfahren ist beispielsweise in den deutschen Auslegeschriften DE 21 37 042 und DE 21 37 043 beschrieben. Man verwendet hierzu eine Vorrichtung, die aus einem zylindrischen Behälter mit stationären, festen Seitenwänden und einer bodenseitig drehbar gelagerten Reibplatte bestehen. Vorrichtungen dieser Art sind unter der Warenbezeichnung Marumerizer^{(R)} in der Technik verbreitet. Nach der Sphäronisierung werden die noch feuchten Kügelchen kontinuierlich oder chargenweise, vorzugsweise unter Verwendung einer Wirbelschichttrockenanlage, bei vorzugsweise 35 °C bis 50 °C und insbesondere bei einer maximalen Produkttemperatur von 45 °C, bis zu einem Restfeuchtegehalt von 4 Gew.-% bis 10 Gew.-%, vorzugsweise 5 Gew.-% bis 8 Gew.-% getrocknet, falls sie vorher höhere Wassergehalte aufweisen. In diesem Verfahrensstadium können durch Sieben oder Windsichten bei der Herstellung des Extrudats eventuell auftretende staubförmige Anteile mit einer Korngröße unter 0,1 mm, insbesondere unter 0,4 mm sowie eventuelle Grobanteile mit einer Korngröße über 2 mm, insbesondere über 1,6 mm entfernt und gegebenenfalls in den Herstellungsprozess zurückgeführt werden. Vorzugsweise wird der Extrusionsprozeß so geführt, daß die entstehenden Mehrenzymextrudate eine derartige Teilchengrößenverteilung besitzen, daß weniger als 10 Gew.-%, insbesondere weniger als 2 Gew.-% der Teilchen einen Durchmesser unter 0,2 mm, 10 Gew.-% bis 20 Gew.-% der Teilchen einen Durchmesser von 0,2 mm bis unter 0,4 mm und 80 Gew.-% bis 90 Gew.-% der Teilchen einen Durchmesser von 0,4 mm bis unter 0,8 mm aufweisen.

Nach oder vorzugsweise während der Trocknung können zusätzlich Stoffe zum Umhüllen und Beschichten der Extrudatpartikel eingebracht werden. Dazu wird der Trocknungsschritt vorzugsweise derart durchgeführt, daß man die die Enzyme enthaltenden Teilchen in einer Wirbelschicht mit einem üblichen Bindemittel, welches in seiner einfachsten Ausführungsform Wasser sein kann, besprüht. Geeignete Bindemittel finden sich auch unter den nichtionischen Tensiden und insbesondere unter den Filmbildnern unter den vorgenannten wasserlöslichen organischen Polymeren, beispielsweise Carboxymethylcellulose und/oder Polyethylenglykol, die in Substanz oder in insbesondere wäßriger Lösung eingesetzt werden können. Weiterhin lassen sich in diesem Stadium auch Farbstoffe oder Pigmente auf die Partikel aufbringen, um so eine eventuelle Eigenfarbe, die meist vom Enzymkonzentrat herrührt, zu überdecken beziehungsweise zu verändern. Als inerte und physiologisch unbedenkliche Pigmente haben sich insbesondere Titandioxid und Calciumcarbonat bewährt, die anschließend oder vorzugsweise zusammen mit dem Bindemittel in wäßriger Dispersion eingebracht werden. Das über die Pigmentdispersion beziehungsweise über das Bindemittel zugeführte Wasser wird bei der gleichzeitig vorgenommenen oder anschließend erneut erforderlichen Trocknung wieder entfernt.

Durch das erfindungsgemäße Verfahren ist es ohne weiteres möglich, Mehrenzymgranulate zu erhalten, die weitgehend die rechnerisch aus der Aktivität der eingesetzten Einzelenzyme zu erwartende Enzymaktivität für jedes enthaltene Enzym aufweisen. In der Regel liegt der Aktivitätserhalt bei über 90 %, insbesondere über 95 % des Erwartungswertes.

Das so erhältliche Mehrenzymgranulat wird vorzugsweise zur Herstellung fester, insbesondere teilchenförmiger Wasch- oder Reinigungsmittel verwendet, die durch einfaches Vermischen der Enzymgranulate mit in derartigen Mitteln üblichen weiteren Komponenten erhalten werden können. Überraschenderweise lassen sich erfindungsgemäße Mehrenzymgranulate aber auch in flüssige beziehungsweise fließfähig-pastenförmige, wasserfreie oder wäßrige Mittel einarbeiten, in denen sich die Mehrenzymgranulate nicht lösen, wobei sich trotz gleichzeitiger Anwesenheit unverträglicher Enzyme eine im Vergleich zu in Lösung eingebrachten Enzymen deutlich erhöhte Enzymlagerstabilität ergibt. Die Viskosität derartiger flüssiger Mittel liegt vorzugsweise im Bereich von 100 mPa.s bis 60 000 mPa.s und läßt sich durch die Einsatzkonzentration beispielsweise von Seife und Lösungsmitteln in breitem Rahmen einstellen. Ein bevorzugter Einsatzbereich insbesondere erfindungsgemäßer Protease-Amylase-Granulate liegt auf dem Gebiet der Geschirreinigungsmittel für die maschinelle Anwendung, welche vorzugsweise als verdichtete Pulver mit erhöhten Schüttgewichten im Bereich von bevorzugt 750 bis 1 000 g/l oder in Tablettenform angeboten werden. Zur Herstellung derartiger Tabletten geht man vorzugsweise derart vor, daß man das Mehrenzymgranulat mit allen weiteren Bestandteilen in einem Mischer vermischt und das Gemisch mittels herkömmlicher Tablettenpressen, beispielsweise Exzenterpressen oder Rundläuferpressen, mit Preßdrucken im Bereich von 200 · 10⁵ Pa bis 1 500 · 10⁵ Pa verpresst. Man erhält so bruchfeste und dennoch unter Anwendungsbedingungen ausreichend rasch lösliche Tabletten mit Biegefestigkeiten von normalerweise über 150 N. Vorzugsweise weist eine derart hergestellte Tablette ein Gewicht von 15 g bis 40 g, insbesondere von 20 g bis 30 g, bei einem Durchmesser von 35 mm bis 40 mm auf.

Für die Einarbeitung in teilchenförmige Wasch- und Reinigungsmittel weist das Mehrenzymgranulat vorzugsweise mittlere Korngrößen im Bereich von 0,9 mm bis 1,8 mm, insbesondere von 1,0 mm bis 1,5 mm auf. Die erfindungsgemäß hergestellten Granulate enthalten vorzugsweise weniger als 5 Gew.-%, insbesondere höchstens 1 Gew.-% an Partikeln mit Korngrößen außerhalb des Bereichs von 0,2 mm bis 1,6 mm.

Die erfindungsgemäß erhaltene Enzymzubereitung besteht aus weitgehend abgerundeten, staubfreien Partikeln, die in der Regel ein Schüttgewicht von etwa 650 bis 1050 Gramm pro Liter, insbesondere 700 bis 880 Gramm pro Liter aufweisen. Die erfindungsgemäß hergestellten Granulate zeichnen sich, insbesondere bei Temperaturen über Raumtemperatur und hoher Luftfeuchtigkeit durch eine sehr hohe Lagerstabilität aus, die, obwohl miteinander reagieren könnende Enzyme vorhanden sind, in der Regel sogar die Lagerstabilität von separat konfektionierten Einzelenzymen überschreitet. Dies gilt sowohl für die erfindungsgemäßen Enzymgranulate als solche wie auch für die in teilchenförmige Wasch- oder Reinigungsmittel eingearbeiteten erfindungsgemäßen Enzymgranulate. Als weiterer Vorteil der erfindungsgemäßen Enzymgranulate ist ihr Löseverhalten unter Anwendungsbedingungen in der Waschflotte zu bemerken, in die sämtliche enthaltenen Enzyme gleichzeitig freigesetzt werden und ihre Reinigungswirkung entfalten können. Vorzugsweise setzen die erfindungsgemäßen Granulate mindestens 90 % ihrer Enzymaktivität innerhalb von 3 Minuten, insbesondere innerhalb von 70 Sekunden bis 2,5 Minuten, in Wasser bei 25 °C frei.

Wasch- oder Reinigungsmittel, die ein erfindungsgemäßes beziehungsweise nach dem erfindungsgemäßen Verfahren hergestelltes Mehrenzymgranulat enthalten, können alle üblichen sonstigen Bestandteile derartiger Mittel enthalten, die nicht in unerwünschter Weise mit den Enzymen wechselwirken. Vorzugsweise wird das Mehrenzymgranulat in Mengen von 0,1 Gew.-% bis 5 Gew.-%, insbesondere 0,5 Gew.-% bis 2,5 Gew.-% in Wasch- oder Reinigungsmittel eingearbeitet.

Überraschenderweise wurde gefunden, daß Enzymgranulate mit den oben angegebenen Eigenschaften die Wirkung bestimmter anderer Wasch- und Reinigungsmittelinhaltsstoffe synergistisch beeinflussen und daß umgekehrt die Wirkung der in den Mehrenzymgranulaten enthaltenen Enzymen durch bestimmte andere Waschmittelinhaltsstoffe synergistisch verstärkt wird. Diese Effekte treten insbesondere bei nichtionischen Tensiden, bei schmutzablösevermögenden Copolyestern, insbesondere solchen mit Terephthalsäureeinheiten, bei wasserunlöslichen anorganischen Buildern, bei wasserlöslichen anorganischen und organischen Buildern, insbesondere auf Basis oxidierter Kohlenhydrate, bei Bleichmitteln auf Persauerstoffbasis, insbesondere bei Alkalipercarbonat, und bei synthetischen Aniontensiden vom Sulfat- und Sulfonattyp, allerdings nicht oder nur wenig ausgeprägt bei Alkylbenzolsulfonaten, auf, weshalb der Einsatz dergenannter Inhaltsstoffe zusammen mit erfindungsgemäßen Mehrenzymgranulaten bevorzugt ist.

In einer bevorzugten Ausführungsform enthält ein solches Mittel nichtionisches Tensid, ausgewählt aus Fettalkylpolyglykosiden, Fettalkylpolyalkoxylaten, insbesondere -ethoxylaten und/oder -propoxylaten, Fettsäurepolyhydroxyamiden und/oder Ethoxylierungs-und/oder Propoxylierungsprodukten von Fettalkylaminen, vicinalen Diolen, Fettsäurealkylestern und/oder Fettsäureamiden sowie deren Mischungen, insbesondere in einer Menge im Bereich von 2 Gew.-% bis 25 Gew.-%.

Eine weitere Ausführungsform derartiger Mittel umfaßt die Anwesenheit von synthetischem Aniontensid vom Sulfat- und/oder Sulfonattyp, insbesondere Fettalkylsulfat, Fettalkylethersulfat, Sulfofettsäureester und/oder Sulfofettsäuredisalze, insbesondere in einer Menge im Bereich von 2 Gew.-% bis 25 Gew.-%. Bevorzugt wird das Aniontensid aus den Alkyl- bzw. Alkenylsulfaten und/oder den Alkyl- bzw. Alkenylethersulfaten ausgewählt, in denen die Alkyl- bzw. Alkenylgruppe 8 bis 22, insbesondere 12 bis 18 C-Atome besitzt.

Zu den in Frage kommenden nichtionischen Tensiden gehören die Alkoxylate, insbesondere die Ethoxylate und/oder Propoxylate von gesättigten oder einbis mehrfach ungesättigten linearen oder verzweigtkettigen Alkoholen mit 10 bis 22 C-Atomen, vorzugsweise 12 bis 18 C-Atomen. Der Alkoxylierungsgrad der Alkohole liegt dabei in der Regel zwischen 1 und 20, vorzugsweise zwischen 3 und 10. Sie können in bekannter Weise durch Umsetzung der entsprechenden Alkohole mit den entsprechenden Alkylenoxiden hergestellt werden. Geeignet sind insbesondere die Derivate der Fettalkohole, obwohl auch deren verzweigtkettige Isomere, insbesondere sogenannte Oxoalkohole, zur Herstellung verwendbarer Alkoxylate eingesetzt werden können. Brauchbar sind demgemäß die Alkoxylate, insbesondere die Ethoxylate, primärer Alkohole mit linearen, insbesondere Dodecyl-, Tetradecyl-, Hexadecyl- oder Octadecyl-Resten sowie deren Gemische. Außerdem sind entsprechende Alkoxylierungsprodukte von Alkylaminen, vicinalen Diolen und Carbonsäureamiden, die hinsichtlich des Alkylteils den genannten Alkoholen entsprechen, verwendbar. Darüberhinaus kommen die Ethylenoxid- und/oder Propylenoxid-Insertionsprodukte von Fettsäurealkylestern, wie sie gemäß dem in der internationalen Patentanmeldung WO 90/13533 angegebenen Verfahren hergestellt werden können, sowie Fettsäurepolyhydroxyamide, wie sie gemäß den Verfahren der US-amerikanischen Patentschriften US 1 985 424, US 2 016 962 und US 2 703 798 sowie der internationalen Patentanmeldung WO 92/06984 hergestellt werden können, in Betracht. Zur Einarbeitung in die erfindungsgemäßen Mittel geeignete sogenannte Alkylpolyglykoside sind Verbindungen der allgemeinen Formel (G)ₙ-OR¹, in der R¹ einen Alkyl- oder Alkenylrest mit 8 bis 22 C-Atomen, G eine Glykoseeinheit und n eine Zahl zwischen 1 und 10 bedeuten. Derartige Verbindungen und ihre Herstellung werden zum Beispiel in den europäischen Patentanmeldungen EP 92 355, EP 301 298, EP 357 969 und EP 362 671 oder der US-amerikanischen Patentschrift US 3 547 828 beschrieben. Bei der Glykosidkomponente (G)ₙ handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad n nimmt als analytisch zu ermittelnde Größe im allgemeinen gebrochene Zahlenwerte an; er liegt bei Werten zwischen 1 und 10, bei den vorzugsweise eingesetzten Glykosiden unter einem Wert von 1,5, insbesondere zwischen 1,2 und 1,4. Bevorzugter Monomer-Baustein ist wegen der guten Verfügbarkeit Glucose. Der Alkyl- oder Alkenylteil R¹ der Glykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Glykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders bevorzugte Alkylglykoside enthalten einen Kokosfettalkylrest, das heißt Mischungen mit im wesentlichen R¹=Dodecyl und R¹=Tetradecyl.

Nichtionisches Tensid ist in Mitteln, welche ein erfindungsgemäßes Mehrenzymgranulat enthalten, vorzugsweise in Mengen von 1 Gew.-% bis 30 Gew.-%, insbesondere von 1 Gew.-% bis 25 Gew.-% enthalten.

Solche Mittel können stattdessen oder zusätzlich weitere Tenside, vorzugsweise synthetische Aniontenside des Sulfat- oder Sulfonat-Typs, in Mengen von vorzugsweise nicht über 20 Gew.-%, insbesondere von 0,1 Gew.-% bis 18 Gew.-%, jeweils bezogen auf gesamtes Mittel, enthalten. Als für den Einsatz in derartigen Mitteln besonders geeignete synthetische Aniontenside sind die Alkyl- und/oder Alkenylsulfate mit 8 bis 22 C-Atomen, die ein Alkali-, Ammonium- oder Alkyl- beziehungsweise Hydroxyalkyl-substituiertes Ammoniumion als Gegenkation tragen, zu nennen. Bevorzugt sind die Derivate der Fettalkohole mit insbesondere 12 bis 18 C-Atomen und deren verzweigtkettiger Analoga, der sogenannten Oxoalkohole. Die Alkyl- und Alkenylsulfate können in bekannter Weise durch Reaktion der entsprechenden Alkoholkomponente mit einem üblichen Sulfatierungsreagenz, insbesondere Schwefeltrioxid oder Chlorsulfonsäure, und anschließende Neutralisation mit Alkali-, Ammonium- oder Alkyl- beziehungsweise Hydroxyalkyl-substituierten Ammoniumbasen hergestellt werden. Derartige Alkyl- und/oder Alkenylsulfate sind in den Mitteln, welche ein erfindungsgemäßes Mehrenzymgranulat enthalten, vorzugsweise in Mengen von 0,1 Gew.-% bis 20 Gew.-%, insbesondere von 0,5 Gew.-% bis 18 Gew.-% enthalten.

Zu den einsetzbaren Tensiden vom Sulfat-Typ gehören auch die sulfatierten Alkoxylierungsprodukte der genannten Alkohole, sogenannte Ethersulfate. Vorzugsweise enthalten derartige Ethersulfate 2 bis 30, insbesondere 4 bis 10, Ethylenglykol-Gruppen pro Molekül. Zu den geeigneten Aniontensiden vom Sulfonat-Typ gehören die durch Umsetzung von Fettsäureestern mit Schwefeltrioxid und anschließender Neutralisation erhältlichen α-Sulfoester, insbesondere die sich von Fettsäuren mit 8 bis 22 C-Atomen, vorzugsweise 12 bis 18 C-Atomen, und linearen Alkoholen mit 1 bis 6 C-Atomen, vorzugsweise 1 bis 4 C-Atomen, ableitenden Sulfonierungsprodukte, sowie die durch formale Verseifung aus diesen hervorgehenden Sulfofettsäuren.

Als weitere fakultative tensidische Inhaltsstoffe kommen Seifen in Betracht, wobei gesättigte Fettsäureseifen, wie die Salze der Laurinsäure, Myristinsäure, Palmitinsäure oder Stearinsäure, sowie aus natürlichen Fettsäuregemischen, zum Beispiel Kokos-, Palmkern- oder Talgfettsäuren, abgeleitete Seifen geeignet sind. Insbesondere sind solche Seifengemische bevorzugt, die zu 50 Gew.-% bis 100 Gew.-% aus gesättigten C₁₂-C₁₈-Fettsäureseifen und zu bis 50 Gew.-% aus Ölsäureseife zusammengesetzt sind. Vorzugsweise ist Seife in Mengen von 0,1 Gew.-% bis 5 Gew.-% enthalten. Insbesondere in flüssigen Mitteln, welche ein erfindungsgemäßes Mehrenzymgranulat enthalten, können jedoch auch höhere Seifenmengen von in der Regel bis zu 20 Gew.-% enthalten sein.

In einer weiteren Ausführungsform enthält ein Mittel, welches ein erfindungsgemäßes Mehrenzymgranulat enthält, wasserlöslichen und/oder wasserunlöslichen Builder, insbesondere ausgewählt aus Alkalialumosilikat, kristallinem Alkalisilikat mit Modul über 1, monomerem Polycarboxylat, polymerem Polycarboxylat und deren Mischungen, insbesondere in Mengen im Bereich von 2,5 Gew.-% bis 60 Gew.-%.

Ein Mittel, welche ein erfindungsgemäßes Mehrenzymgranulat enthält, enthält vorzugsweise 20 Gew.-% bis 55 Gew.-% wasserlöslichen und/oder wasserunlöslichen, organischen und/oder anorganischen Builder. Zu den wasserlöslichen organischen Buildersubstanzen gehören insbesondere solche aus der Klasse der Polycarbonsäuren, insbesondere Citronensäure und Zuckersäuren, sowie der polymeren (Poly-)carbonsäuren, insbesondere die durch Oxidation von Polysacchariden zugänglichen Polycarboxylate der internationalen Patentanmeldung WO 93/16110, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5000 und 200000, die der Copolymeren zwischen 2000 und 200000, vorzugsweise 50000 bis 120000, bezogen auf freie Säure. Ein besonders bevorzugtes Acrylsäure-Maleinsäure-Copolymer weist eine relative Molekülmasse von 50000 bis 100000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei Carbonsäuren und/oder deren Salze sowie als drittes Monomer Vinylalkohol und/oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth-)acrylsäure ab. Das zweite saure Monomer beziehungsweise dessen Salz kann ein Derivat einer C₄-C₈-Dicarbonsäure, vorzugsweise einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Terpolymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Terpolymere, in denen das Gewichtsverhältnis (Meth)acrylsäure beziehungsweise (Meth)acrylat zu Maleinsäure beziehungsweise Maleat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di-oder Oligosaccharide bevorzugt sind, besonders bevorzugt ist Saccharose. Durch den Einsatz des dritten Monomers werden vermutlich Sollbruchstellen in dem Polymer eingebaut, die für die gute biologische Abbaubarkeit des Polymers verantwortlich sind. Diese Terpolymere lassen sich insbesondere nach Verfahren herstellen, die in der deutschen Patentschrift DE 42 21 381 und der deutschen Patentanmeldung P 43 00 772.4 beschrieben sind, und weisen im allgemeinen eine relative Molekülmasse zwischen 1000 und 200000, vorzugsweise zwischen 200 und 50000 und insbesondere zwischen 3000 und 10000 auf. Sie können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Polycarbonsäuren werden in der Regel in Form ihrer wasserlöslichen Salze, insbesondere ihre Alkalisalze, eingesetzt.

Derartige organische Buildersubstanzen sind vorzugsweise in Mengen bis zu 40 Gew.-%, insbesondere bis zu 25 Gew.-% und besonders bevorzugt von 1 Gew.-% bis 5 Gew.-% enthalten. Mengen nahe der genannten Obergrenze werden vorzugsweise in pastenförmigen oder flüssigen, insbesondere wasserhaltigen, Mitteln eingesetzt, in welchen das erfindungsgemäße Mehrenzymgranulat enthalten ist.

Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Alumosilikate in Waschmittelqualität, insbesondere Zeolith NaA und gegebenenfalls NaX, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt im Bereich von 100 bis 200 mg CaO pro Gramm. Geeignete Substitute beziehungsweise Teilsubstitute für das genannte Alumosilikat sind kristalline Alkalisilikate, die allein oder im Gemisch mit amorphen Silikaten vorliegen können. Die in den Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Derartige amorphe Alkalisilikate sind beispielsweise unter dem Namen Portil^{(R)} im Handel erhältlich. Solche mit einem molaren Verhältnis Na₂O:SiO₂ von 1:1,9 bis 1:2,8 können nach dem Verfahren der europäischen Patentanmeldung EP 0 425 427 hergestellt werden. Sie werden im Rahmen der Herstellung bevorzugt als Feststoff und nicht in Form einer Lösung zugegeben. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·yH₂O eingesetzt, in der x, das sogenannte Modul, eine Zahl von 1,9 bis 4 und y eine Zahl von 0 bis 20 ist und bevorzugte Werte für x 2, 3 oder 4 sind. Kristalline Schichtsilikate, die unter diese allgemeine Formel fallen, werden beispielsweise in der europäischen Patentanmeldung EP 0 164 514 beschrieben. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅.yH₂O) bevorzugt, wobei β-Natriumdisilikat beispielsweise nach dem Verfahren erhalten werden kann, das in der internationalen Patentanmeldung WO 91/08171 beschrieben ist. δ-Natriumsilikate mit einem Modul zwischen 1,9 und 3,2 können gemäß den japanischen Patentanmeldungen JP 04/238 809 oder JP 04/260 610 hergestellt werden. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, herstellbar wie in den europäischen Patentanmeldungen EP 0 548 599, EP 0 502 325 und EP 0 452 428 beschrieben, können in Mitteln, welche ein erfindungsgemäßes Mehrenzymgranulat enthalten, eingesetzt werden. In einer weiteren bevorzugten Ausführungsform erfindungsgemäßer Mittel wird ein kristallines Natriumschichtsilikat mit einem Modul von 2 bis 3 eingesetzt, wie es nach dem Verfahren der europäischen Patentanmeldung EP 0 436 835 aus Sand und Soda hergestellt werden kann. Kristalline Natriumsilikate mit einem Modul im Bereich von 1,9 bis 3,5, wie sie nach den Verfahren der europäischen Patentschriften EP 0 164 552 und/oder EP 0 293 753 erhältlich sind, werden in einer weiteren bevorzugten Ausführungsform von Wasch- oder Reinigungsmitteln, welche ein erfindungsgemäßes Mehrenzymgranulat enthalten, eingesetzt. Deren Gehalt an Alkalisilikaten beträgt vorzugsweise 1 Gew.-% bis 50 Gew.-% und insbesondere 5 Gew.-% bis 35 Gew.-%, bezogen auf wasserfreie Aktivsubstanz. Falls als zusätzliche Buildersubstanz auch Alkalialumosilikat, insbesondere Zeolith, vorhanden ist, beträgt der Gehalt an Alkalisilikat vorzugsweise 1 Gew.-% bis 15 Gew.-% und insbesondere 2 Gew.-% bis 8 Gew.-%, bezogen auf wasserfreie Aktivsubstanz. Das Gewichtsverhältnis Alumosilikat zu Silikat, jeweils bezogen auf wasserfreie Aktivsubstanzen, beträgt dann vorzugsweise 4:1 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Zusätzlich zum genannten anorganischen Builder können weitere wasserlösliche oder wasserunlösliche anorganische Substanzen in den Mitteln, welche ein erfindungsgemäßes Mehrenzymgranulat enthalten, eingesetzt werden. Geeignet sind in diesem Zusammenhang die Alkalicarbonate, Alkalihydrogencarbonate und Alkalisulfate sowie deren Gemische. Derartiges zusätzliches anorganisches Material kann in Mengen bis zu 70 Gew.-% vorhanden sein, fehlt jedoch vorzugsweise ganz.

Zusätzlich können die Mittel weitere in Wasch- und Reinigungsmitteln übliche Bestandteile enthalten. Zu diesen fakultativen Bestandteilen gehören insbesondere Bleichmittel, Bleichaktivatoren, Komplexbildner für Schwermetalle, beispielsweise Aminopolycarbonsäuren, Aminohydroxypolycarbonsäuren, Polyphosphonsäuren und/oder Aminopolyphosphonsäuren, Vergrauungsinhibitoren, beispielsweise Celluloseether, Farbübertragungsinhibitoren, beispielsweise Polyvinylpyrrolidon oder Polyvinylpyrdin-N-oxid, Schauminhibitoren, beispielsweise Organopolysiloxane oder Paraffine, Lösungsmittel und optische Aufheller, beispielsweise Stilbendisulfonsäurederivate. Vorzugsweise sind in Mitteln, welche ein erfindungsgemäßes Mehrenzymgranulat enthalten, bis zu 1 Gew.-%, insbesondere 0,01 Gew.-% bis 0,5 Gew.-% optische Aufheller, insbesondere Verbindungen aus der Klasse der substituierten 4,4'-Bis-(2,4,6-triamino-s-triazinyl)-stilben-2,2'-disulfonsäuren, bis zu 5 Gew.-%, insbesondere 0,1 Gew.-% bis 2 Gew.-% Komplexbildner für Schwermetalle, insbesondere Aminoalkylenphosphonsäuren und deren Salze, bis zu 3 Gew.-%, insbesondere 0,5 Gew.-% bis 2 Gew.-% Vergrauungsinhibitoren und bis zu 2 Gew.-%, insbesondere 0,1 Gew.-% bis 1 Gew.-% Schauminhibitoren enthalten, wobei sich die genannten Gewichtsanteile jeweils auf gesamtes Mittel beziehen.

Lösungsmittel, die insbesondere bei flüssigen Mitteln, welche ein erfindungsgemäßes Mehrenzymgranulat enthalten, eingesetzt werden, und die auch im Rahmen des Herstellungsverfahrens der erfindungsgemäßen Mehrenzymgranulate im flüssigen Primär- und/oder Sekundärenzym vorhanden sein können, sind neben Wasser vorzugsweise solche, die wassermischbar sind. Zu diesen gehören die niederen Alkohole, beispielsweise Ethanol, Propanol, iso-Propanol, und die isomeren Butanole, Glycerin, niedere Glykole, beispielsweise Ethylen- und Propylenglykol, und die aus den genannten Verbindungsklassen ableitbaren Ether. In derartigen flüssigen Mitteln liegen die Mehrenzymgranulate ungelöst, daß heißt in fester, granularer Form, vor.

Zu den gegebenenfalls, insbesondere in flüssigen erfindungsgemäßen Mitteln, vorhandenen üblichen Enzymstabilisatoren gehören Aminoalkohole, beispielsweise Mono-, Di-, Triethanol- und -propanolamin und deren Mischungen, niedere Carbonsäuren, wie beispielsweise aus den europäischen Patentanmeldungen EP 376 705 und EP 378 261 bekannt, Borsäure beziehungsweise Alkaliborate, Borsäure-Carbonsäure-Kombinationen, wie beispielsweise aus der europäischen Patentanmeldung EP 451 921 bekannt, Borsäureester, wie beispielsweise aus der internationalen Patentanmeldung WO 93/11215 oder der europäischen Patentanmeldung EP 511 456 bekannt, Boronsäurederivate, wie beispielsweise aus der europäischen Patentanmeldung EP 583 536 bekannt, Calciumsalze, beispielsweise die aus der europäischen Patentschrift EP 28 865 bekannte Ca-Ameisensäure-Kombination, Magnesiumsalze, wie beispielsweise aus der europäischen Patentanmeldung EP 378 262 bekannt, und/ oder schwefelhaltige Reduktionsmittel, wie beispielsweise aus den europäischen Patentanmeldungen EP 080 748 oder EP 080 223 bekannt.

Zu den geeigneten Schauminhibitoren gehören langkettige Seifen, insbesondere Behenseife, Fettsäureamide, Paraffine, Wachse, Mikrokristallinwachse, Organopolysiloxane und deren Gemische, die darüberhinaus mikrofeine, gegebenenfalls silanierte oder anderweitig hydrophobierte Kieselsäure enthalten können. Zum Einsatz in partikelförmigen Mitteln sind derartige Schauminhibitoren vorzugsweise an granulare, wasserlösliche Trägersubstanzen gebunden, wie beispielsweise in der deutschen Offenlegungsschrift DE 34 36 194, den europäischen Patentanmeldungen EP 262 588, EP 301 414, EP 309 931 oder der europäischen Patentschrift EP 150 386 beschrieben.

Ferner kann das ein Mittel, welches ein erfindungsgemäßes Mehrenzymgranulat enthält, Vergrauungsinhibitoren enthalten. Vergrauungsinhibitoren haben die Aufgabe, den von der Faser abgelösten Schmutz in der Flotte suspendiert zu halten und so das Vergrauen der Fasern zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, zum Beispiel teilhydrolysierte Stärke. Na-Carboxymethylcellulose, Methylcellulose, Methylhydroxyethylcellulose und deren Gemische werden bevorzugt eingesetzt.

Eine weitere Ausführungsform eines derartigen Mittels, welches ein erfindungsgemäßes Mehrenzymgranulat enthält, enthält Bleichmittel auf Persauerstoffbasis, insbesondere in Mengen im Bereich von 5 Gew.-% bis 70 Gew.-%, sowie gegebenenfalls Bleichaktivator, insbesondere in Mengen im Bereich von 2 Gew.-% bis 10 Gew.-%. Diese in Betracht kommenden Bleichmittel sind die in Waschmitteln in der Regel verwendeten Perverbindungen wie Wasserstoffperoxid, Perborat, das als Tetra- oder Monohydrat vorliegen kann, Percarbonat, Perpyrophosphat und Persilikat, die in der Regel als Alkalisalze, insbesondere als Natriumsalze, vorliegen. Derartige Bleichmittel sind in Waschmitteln, welche ein erfindungsgemäßes Mehrenzymgranulat enthalten, vorzugsweise in Mengen bis zu 25 Gew.-%, insbesondere bis zu 15 Gew.-% und besonders bevorzugt von 5 Gew.-% bis 15 Gew.-%, jeweils bezogen auf gesamtes Mittel, vorhanden. Die fakultativ vorhandene Komponente der Bleichaktivatoren umfaßt die üblicherweise verwendeten N- oder 0-Acylverbindungen, beispielsweise mehrfach acylierte Alkylendiamine, insbesondere Tetraacetylethylendiamin, acylierte Glykolurile, insbesondere Tetraacetylglykoluril, N-acylierte Hydantoine, Hydrazide, Triazole, Urazole, Diketopiperazine, Sulfurylamide und Cyanurate, außerdem Carbonsäureanhydride, insbesondere Phthalsäureanhydrid, Carbonsäureester, insbesondere Natrium-isononanoyl-phenolsulfonat, und acylierte Zuckerderivate, insbesondere Pentaacetylglukose. Die Bleichaktivatoren können zur Vermeidung der Wechselwirkung mit den Perverbindungen bei der Lagerung in bekannter Weise mit Hüllsubstanzen überzogen beziehungsweise granuliert worden sein, wobei mit Hilfe von Carboxymethylcellulose granuliertes Tetraacetylethylendiamin mit mittleren Korngrößen von 0,01 mm bis 0,8 mm, wie es beispielsweise nach dem in der europäischen Patentschrift EP 037 026 beschriebenen Verfahren hergestellt werden kann, und/oder granuliertes 1,5-Diacetyl-2,4-dioxohexahydro-1,3,5-triazin, wie es nach dem in der deutschen Patentschrift DD 255 884 beschriebenen Verfahren hergestellt werden kann, besonders bevorzugt ist. In Waschmitteln sind derartige Bleichaktivatoren vorzugsweise in Mengen bis zu 8 Gew.-%, insbesondere von 2 Gew.-% bis 6 Gew.-%, jeweils bezogen auf gesamtes Mittel, enthalten.

Schließlich umfaßt eine weitere Ausführungsform eines Mittels, welches ein erfindungsgemäßes Mehrenzymgranulat enthält, die Anwesenheit von schmutzablösevermögenden Substanzen auf Basis von Copolyestern aus Dicarbonsäuren und Glykolen, die insbesondere in Mengen von 0,01 Gew.-% bis 5 Gew.-% enthalten sein können. Oft als "soil-release"-Wirkstoffe bezeichnete schmutzablösevermögende Substanzen, die wegen ihrer chemischen Ähnlichkeit zu Polyesterfasern besonders wirksam sind, aber auch bei Geweben aus anderem Material die erwünschte Wirkung zeigen können, sind Copolyester, die Dicarbonsäureeinheiten, Alkylenglykoleinheiten und Polyalkylenglykoleinheiten enthalten. Schmutzablösevermögende Copolyester der genannten Art wie auch ihr Einsatz in Waschmitteln sind seit langer Zeit bekannt. So beschreibt zum Beispiel die deutsche Offenlegungsschrift DT 16 17 141 ein Waschverfahren unter Einsatz von Polyethylenterephthalat-Polyoxyethylenglykol-Copolymeren. Die deutsche Offenlegungsschrift DT 22 00 911 betrifft Waschmittel, die Niotensid und ein Mischpolymer aus Polyoxyethylenglykol und Polyethylenterephthalat enthalten. In der deutschen Offenlegungsschrift DT 22 53 063 sind saure Textilausrüstungsmittel genannt, die ein Copolymer aus einer dibasigen Carbonsäure und einem Alkylen- oder Cycloalkylenpolyglykol sowie gegebenenfalls einem Alkylen- oder Cycloalkylenglykol enthalten. Das europäische Patent EP 066 944 betrifft Textilbehandlungsmittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 185 427 sind Methyl- oder Ethylgruppen-endverschlossene Polyester mit Ethylen-und/oder Propylen-terephthalat- und Polyethylenoxid-terephthalat-Einheiten und Waschmitel, die derartiges Soil-release-Polymer enthalten, bekannt. Das europäische Patent EP 241 984 betrifft einen Polyester, der neben Oxyethylen-Gruppen und Terephthalsäureeinheiten auch substituierte Ethyleneinheiten sowie Glycerineinheiten enthält. Derartige soil-release-Polyester sind in Mitteln, die ein erfindungsgemäßes Mehrenzymgranulat enthalten, vorzugsweise in Mengen von 0,1 Gew.-% bis 2,5 Gew.-%, insbesondere von 0,2 Gew.-% bis 2 Gew.-% enthalten.

Zusätzlich kann ein derartiges Wasch- oder Reinigungsmittel auch Enzyme enthalten, die separat zugefügt werden müssen, das heißt nicht über das erfindungsgemäße Mehrenzymgranulat eingearbeitet werden. Vorzugsweise sind in solchen Mitteln jedoch sämtliche enthaltenen Enzyme in Form des Mehrenzymgranulats vorhanden.

In einer bevorzugten Ausführungsform ist ein Mittel, in das erfindungsgemäßes beziehungsweise erfindungsgemäßhergestelltes Mehrenzymgranulat eingearbeitet wird, teilchenförmig und enthält 20 Gew.-% bis 55 Gew.-% anorganischen Builder, bis zu 15 Gew.-%, insbesondere 2 Gew.-% bis 12 Gew.-% wasserlöslichen organischen Builder, 2,5 Gew.-% bis 20 Gew.-% synthetisches Aniontensid, 1 Gew.-% bis 20 Gew.-% nichtionisches Tensid, bis zu 25 Gew.-%, insbesondere 1 Gew.-% bis 15 Gew.-% Bleichmittel, bis zu 8 Gew.-%, insbesondere 0,5 Gew.-% bis 6 Gew.-% Bleichaktivator und bis zu 20 Gew.-%, insbesondere 0,1 Gew.-% bis 15 Gew.-% anorganische Salze, insbesondere Alkalicarbonat und/oder -sulfat.

In einer weiteren bevorzugten Ausführungsform enthält ein derartiges pulverförmiges Mittel, insbesondere zur Verwendung als Feinwaschmittel, 20 Gew.-% bis 55 Gew.-% anorganischen Builder, bis zu 15 Gew.-%, insbesondere 2 Gew.-% bis 12 Gew.-% wasserlöslichen organischen Builder, 4 Gew.-% bis 24 Gew.-% nichtionisches Tensid, bis zu 15 Gew.-%, insbesondere 1 Gew.-% bis 10 Gew.-% synthetisches Aniontensid, bis zu 65 Gew.-%, insbesondere 1 Gew.-% bis 30 Gew.-% anorganische Salze, insbesondere Alkalicarbonat und/oder -sulfat, und weder Bleichmittel noch Bleichaktivator.

Eine weitere bevorzugte Ausführungsform umfaßt ein flüssiges Mittel, enthaltend 5 Gew.-% bis 35 Gew.-% wasserlöslichen organischen Builder, bis zu 15 Gew.-%, insbesondere 0,1 Gew.-% bis 5 Gew.-% wasserunlöslichen anorganischen Builder, bis zu 15 Gew.-%, insbesondere 0,5 Gew.-% bis 10 Gew.-% synthetisches Aniontensid, 1 Gew.-% bis 25 Gew.-% nichtionisches Tensid, bis zu 15 Gew.-%, insbesondere 4 Gew.-% bis 12 Gew.-% Seife und bis zu 30 Gew.-% , insbesondere 1 Gew.-% bis 25 Gew.-% Wasser und/oder wassermischbares Lösungsmittel.

### Beispiele

### Beispiel 1

Durch Fermentation von nach dem in der internationalen Patentanmeldung WO 91/02792 beschriebenen Verfahren durch Transformation einer Gensequenz aus Bacillus lentus DSM 5483 modifiziertem Bacillus licheniformis (ATCC 53926) analog dem in der deutschen Patentschrift DE 29 25 427 angegebenen Verfahren wurde eine biomassehaltige Fermenterbrühe erhalten, die ca. 65 000 Proteaseeinheiten pro Gramm (PE/g) enthielt. Diese wurde durch Dekantieren, Querstrom-Mikrofiltration, Ultrafiltation (Trenngrenze bei Molekulargewicht 10 000) und anschließendes Eindampfen im Vakuum gemäß dem in der internationalen Patentanmeldung WO 92/11347 beschriebenen Vorgehen zu einem Proteasegehalt von 700 000 PE/g aufkonzentriert. Dieser setzte man 50 Gew.-Teile Propylenglykol als Inhibitor zu. Anschließend wurde die Primärenzymlösung mit den in Tabelle 1 angegebenen handelsüblichen flüssigen Enzymformulierungen vermischt. Zu dieser Enzymmischung wurden in einem mit rotierendem Schlagwerkzeug ausgerüsteten Mischer 45 Gew.-Teile Cellulosepulver (Technocel^{(R)} 30, Hersteller Cellulose Füllstoff Fabrik), 35 Gew.-Teile Saccharose, 120 Gew.-Teile Na-Carboxymethylcellulose (Tylose^{(R)}, Hersteller Hoechst), 50 Gew.-Teile Polyethylenglykol (mittleres Molgewicht 2000), 300 Gew.-Teile Maisstärke und 130 Gew.-Teile Weizenmehl zugemischt und in einem mit einer Außenkühlung versehenen Kneter homogenisiert. Die Extrusion der plastischen Masse erfolgte mit Hilfe eines mit einer Lochscheibe (Lochdurchmesser 0,8 mm) und einem rotierenden Messer ausgerüsteten Extruders. Man erhielt die in Tabelle durch ihre Enzymzusammensetzung charakterisierten Enzymextrudate mit Längen von jeweils 0,8 mm, die mit 3 Gew.-Teilen Calciumcarbonat abgepudert und in einer Sphäronisierungsvorrichtung (Marumerizer^{(R)}) während einer Bearbeitungszeit von etwa 1 Minute zu gleichmäßig abgerundeten Partikeln verformt und entgratet wurden. Das den Sphäronisator verlassende Gut wurde in einem Wirbelschichttrockner bei Temperaturen von 40 °C bis 45 °C getrocknet und mit 150 Gew.-Teilen Coatingmaterial, bestehend aus TiO₂, Stearylalkohol und 40-fach ethoxyliertem Ricinusöl, umhüllt. Durch anschließendes Sieben wurden Partikel mit Teilchengrößen unter 0,4 mm und über 1,6 mm (Mengenanteil unter 1 Gew.-%) entfernt.

**Tabelle 1:**

| Enzymgehalt der Extrudate [Gew.-Teile] | | | | |
|---|---|---|---|---|
| | **E1** | **E2** | **E3** | **E4** |
| Protease-Brühe | 190 | 190 | 300 | 190 |
| Amylase^{a)} | 140 | - | 20 | - |
| Lipase^{b)} | - | 140 | - | - |
| Cellulase^{c)} | - | - | - | 140 |

| | | | | |
|---|---|---|---|---|
| ^{a)}: Termamyl^{(R)} 300 L (Flüssigformulierung; Hersteller Novo Nordisk) | | | | |
| ^{b)}: Lipolase^{(R)} 100 L (Flüssigformulierung; Hersteller Novo Nordisk) | | | | |
| ^{c)}: Celluzyme^{(R)} 700 L (Flüssigformulierung; Hersteller Novo Nordisk) | | | | |

### Beispiel 2

Das in Beispiel 1 hergestellte Mehrenzymgranulat E2 wurde in einer Menge von 1 Gew.-Teil mit 99 Gew.-Teilen eines gemäß WO 91/02047 hergestellten Waschmittels mit einem Schüttgewicht von 780 g/l, enthaltend 18 Gew.-% Natriumalkylbenzolsulfonat, 3 Gew.-% Niotensid (Dehydol^{(R)}), 16 Gew.-% Natriumperborat, 29 Gew.-% Zeolith Na-A, 5 Gew.-% Natriumcarbonat, 5 Gew.-% polymeres Polycarboxyalat (Sokalan CP 5, Hersteller BASF), 6 Gew.-% Tetraacetylethylendiamin, 3 Gew.-% Plastifizierhilfsmittel (40-fach ethoxylierter Fettalkohol) und als Rest auf 100 Gew.-% Wasser, vermischt (Mittel **W1**). Zum Vergleich wurde eine Mischung **V1** hergestellt, die den gleichen Anteil des Waschmittels und der Enzyme enthielt, aber in dem die beiden Enzyme verteilt auf zwei separate Partikel (Proteasegranulat gemäß internationaler Patentanmeldung WO 92/11347; Lipolase^{(R)} 100 T, Hersteller Novo Nordisk) vorlagen. Die beiden Mittel wurden über 6 Wochen bei 40 °C und 80 % relativer Luftfeuchtigkeit gelagert. In der nachfolgenden Tabelle 2 sind die Enzymaktivitäten (in willkürlichen Einheiten) vor und nach Lagerung angegeben. Man erkennt, daß im das erfindungsgemäße Mehrenzymgranulat enthaltenden Waschmittel die Stabilität der in einem Partikel vorliegenden Enzyme signifikant höher ist als wenn die Enzyme in getrennten Partikeln konfektioniert vorliegen. Der gleiche überraschende Befund trifft auch auf die Mehrenzymextrudate **E1**, **E3** und **E4** des Beispiels 1 zu.

**Tabelle 2:**

| Enzymaktivitäten | | |
|---|---|---|
| | Protease-Aktivität | Lipase-Aktivität |
| **W1**: Start | 142 | 38 |
| Lagerung 6 Wochen | 136 | 34 |
| **V1**: Start | 117 | 75 |
| Lagerung 6 Wochen | 75 | 52 |

## Patentansprüche

1. Verfahren zur Herstellung von Enzymgranulaten, die mindestens zwei verschiedene Enzyme enthalten, durch Vermischen einer wäßrigen, ein erstes Enzym enthaltenden Flüssigkeit, die gegegebenfalls eine von unlöslichen Bestandteilen befreite und aufkonzentrierte Fermentationsbrühe sein kann, mit einem kompetitiven Inhibitor für dieses Enzym, anschließendes Vermischen des Primärenzyms mit dem zweiten Enzym beziehungsweise weiteren Enzymen, Zumischen von organischem und/oder anorganischem Trägermaterial, Extrusion der so erhaltenen Mischung aus Enzymen und Zuschlagstoffen durch eine Lochplatte mit anschließendem Schneidegerät, gegebenenfalls Sphäronisierung des Extrudats in einem Rondiergerät sowie Trocknung, und gegebenenfalls Aufbringen eines gewünschtenfalls Farbstoff und/oder Pigment enthaltenden Überzugs.

2. Verfahren nach Anspruch 1, dadurch gekennzeichnet, daß die das Primärenzym enthaltende Flüssigkeit eine gegegebenfalls von unlöslichen Bestandteilen befreite und aufkonzentrierte wäßrige Fermentationsbrühe ist.

3. Verfahren nach Anspruch 1 oder 2, dadurch gekennzeichnet, daß das Primärenzym Protease ist und das Sekundärenzym aus Amylase, Lipase, Cellulase, Hemicellulase, Oxidase, Peroxidase sowie deren Gemischen ausgewählt wird.

4. Verfahren nach einem der Ansprüche 1 bis 3, dadurch gekennzeichnet, daß man als Primärenzym Protease in solchen Mengen einsetzt, daß das erhaltene Mehrenzymgranulat eine Proteaseaktivität von 50 000 PE/g bis 350 000 PE/g, insbesondere 100 000 PE/g bis 250 000 PE/g aufweist.

5. Verfahren nach einem der Ansprüche 1 bis 4, dadurch gekennzeichnet, daß man als Sekundärenzym Amylase in solchen Mengen einsetzt, daß das erhaltene Mehrenzymgranulat eine Amylaseaktivität von 1 KNU/g bis 100 KNU/g, insbesondere 2 KNU/g bis 60 KNU/g, Lipase in solchen Mengen einsetzt, daß das erhaltene Mehrenzymgranulat eine Lipaseaktivität von 1 KLU/g bis 80 KLU/g, insbesondere 1,5 KLU/g bis 60 KLU/g und/oder Cellulase in solchen Mengen einsetzt, daß das erhaltene Mehrenzymgranulat eine Cellulaseaktivität von 50 CEVU/g bis 1250 CEVU/g und insbesondere 100 CEVU/g bis 1000 CEVU/g aufweist.

6. Verfahren nach einem der Ansprüche 1 bis 5, dadurch gekennzeichnet, daß die Trägermaterialien aus Cellulose, Maltodextrose, Saccharose, Invertzucker, Glukose, Stärken, Getreidemehle, Celluloseether, Alkalialumosilikat, insbesondere Zeolith, Schichtsilikat, insbesondere Bentonit oder Smectit, und wasserlösliche anorganische oder organische Salze, insbesondere Alkalichlorid, Alkalisulfat, Alkalicarbonat oder Alkaliacetat, ausgewählt werden.

7. Verfahren nach einem der Ansprüche 1 bis 6, dadurch gekennzeichnet, daß das Trägermaterial Stärke in Mengen von 20 Gew.-% bis 80 Gew.-%, insbesondere von 25 Gew.-% bis 75 Gew.-%, und Getreidemehl in Mengen von 10 Gew.-% bis 35 Gew.-%, insbesondere von 15 Gew.-% bis 20 Gew.-% jeweils bezogen auf gesamtes Trägermaterial, enthält.

8. Verfahren nach Anspruch 7, dadurch gekennzeichnet, daß im Trägermaterial die Summe der Mengen der Stärke und des Mehls nicht über 95 Gew.-%, insbesondere 60 Gew.-% bis 95 Gew.-% beträgt.

9. Verfahren nach einem der Ansprüche 1 bis 8, dadurch gekennzeichnet, daß das Sekundärenzym anorganisches Salz, insbesondere Alkalisulfat und/oder -chlorid, in Mengen von 30 Gew.-% bis 80 Gew.-%, faser- oder pulverförmige Cellulose in Mengen von 2 Gew.-% bis 40 Gew.-%, und Bindemittel, insbesondere Dextrose, Saccharose, Polyvinylalkohol und/ oder Polyvinylpyrrolidon, in Mengen von 0,1 Gew.-% bis 15 Gew.-% enthält.

10. Verfahren nach einem der Ansprüche 1 bis 9, dadurch gekennzeichnet, daß man nach oder insbesondere während der Trocknung Stoffe zum Umhüllen und Beschichten der Extrudatpartikel einbringt, die gegebenenfalls Farbstoffe oder Pigmente, insbesondere Titandioxid und/oder Calciumcarbonat, umfassen.

11. Verwendung eines Enzymgranulats, erhältlich nach dem Verfahren eines der Ansprüche 1 bis 10, zur Herstellung von Wasch- oder Reinigungsmitteln.

## Claims

1. A process for the production of enzyme granules containing at least two different enzymes by mixing an aqueous liquid containing a first enzyme, which may optionally be a fermentation broth freed from insoluble constituents and concentrated, with a competitive inhibitor for this enzyme, subsequently mixing the primary enzyme with the second enzyme or with further enzymes, incorporating an organic and/or inorganic carrier material, extruding the resulting mixture of enzymes and additives through a multiple-bore die followed by a cutting unit, optionally spheronizing the extrudate in a spheronizing unit and drying and, if desired, applying an optionally dye-and/or pigment-containing coating.

2. A process as claimed in claim 1, characterized in that the liquid containing the primary enzyme is an aqueous fermentation broth optionally freed from insoluble constituents and concentrated.

3. A process as claimed in claim 1 or 2, characterized in that the primary enzyme is protease and the secondary enzyme is selected from amylase, lipase, cellulase, hemicellulase, oxidase, peroxidase and mixtures thereof.

4. A process as claimed in any of claims 1 to 3, characterized in that protease is present as the primary enzyme in such quantities that the multi-enzyme granules obtained have a protease activity of 50,000 PU/g to 350,000 PU/g and, more particularly, in the range from 100,000 PU/g to 250,000 PU/g.

5. A process as claimed in any of claims 1 to 4, characterized in that the secondary enzyme used is amylase in such quantities that the multi-enzyme granules obtained have an amylase activity of 1 KNU/g to 100 KNU/g and, more particularly, from 2 KNU/g to 60 KNU/g, lipase in such quantities that the multi-enzyme granules obtained have a lipase activity of 1 KLU/g to 80 KLU/g and, more particularly, from 1.5 KLU/g to 60 KLU/g and/or cellulase in such quantities that the multi-enzyme granules obtained have a cellulase activity of 50 CEVU/g to 1250 CEVU/g and, more particularly, from 100 CEVU/g to 1000 CEVU/g.

6. A process as claimed in any of claims 1 to 5, characterized in that the carrier materials are selected from cellulose, maltodextrose, sucrose, invert sugar, glucose, starches, cereal flours, cellulose ethers, alkali metal alumosilicate, more particularly zeolite, layer silicate, more particularly bentonite or smectite, and water-soluble inorganic or organic salts, more particularly alkali metal chloride, alkali metal sulfate, alkali metal carbonate or alkali metal acetate.

7. A process as claimed in any of claims 1 to 6, characterized in that the carrier material contains starch in quantities of 20% by weight to 80% by weight and, more particularly, in quantities of 25% by weight to 75% by weight and cereal flour in quantities of 10% by weight to 35% by weight and, more particularly, in quantities of 15% by weight to 20% by weight, based on the carrier material as a whole.

8. A process as claimed in claim 7, characterized in that the sum total of starch and flour in the carrier material is not more than 95% by weight and, more particularly, is between 60% by weight and 95% by weight.

9. A process as claimed in any of claims 1 to 8, characterized in that the secondary enzyme contains inorganic salt, more particularly alkali metal sulfate and/or chloride, in quantities of 30% by weight to 80% by weight, fibrous or powder-form cellulose in quantities of 2% by weight to 40% by weight and binder, more particularly dextrose, sucrose, polyvinyl alcohol and/or polyvinyl pyrrolidone, in quantities of 0.1% by weight to 15% by weight.

10. A process as claimed in any of claims 1 to 9, characterized in that substances for encapsulating and coating the extrudate particles, optionally including dyes or pigments, more particularly titanium dioxide and/or calcium carbonate, are introduced after or, more particularly, during the drying step.

11. The use of the enzyme granules obtainable by the process claimed in any of claims 1 to 10 for the production of detergents or cleaning products.

## Revendications

1. Procédé de production de granulés d'enzymes, qui renferment au moins deux enzymes différentes, par mélangeage d'un liquide aqueux contenant une première enzyme, qui peut être éventuellement un bouillon de fermentation débarrassé des constituants insolubles et concentré, avec un inhibiteur compétitif pour cette enzyme, mélangeage postérieur de l'enzyme primaire avec la deuxième enzyme ou avec des enzymes supplémentaires, addition par mélangeage d'une matière porteuse organique et/ou inorganique, extrusion du mélange d'enzymes et d'agrégats ainsi obtenu par un disque perforé avec un dispositif de coupe intercalé à la suite, éventuellement sphéronisation du produit d'extrusion dans un appareil à arrondir, ainsi que séchage et éventuellement application d'un revêtement renfermant si on le souhaite un colorant et/ou un pigment.

2. Procédé selon la revendication 1, caractérisé en ce que le liquide renfermant l'enzyme primaire est un bouillon de fermentation aqueux, éventuellement débarrassé des constituants insolubles et concentré.

3. Procédé selon la revendication 1 ou 2, caractérisé en ce que l'enzyme primaire est la protéase et l'enzyme secondaire est sélectionnée parmi l'amylase, la lipase, la cellulase, l'hémicellulase, l'oxydase, la peroxydase ainsi que les mélanges de celles-ci.

4. Procédé selon une des revendications 1 à 3, caractérisé en ce que l'on met en oeuvre comme enzyme primaire, de la protéase en proportions telles que le granulé d'enzymes multiples obtenu présente une activité de protéase de 50 000 à 350 000 UP/g, en particulier de 100 000 à 250 000 UP/g.

5. Procédé selon une des revendications 1 à 4, caractérisé en ce que l'on met en oeuvre comme enzyme secondaire, de l'amylase en proportions telles que le granulé d'enzymes multiples obtenu présente une activité d'amylase de 1 à 100 KNU/g, en particulier de 2 à 60 KNU/g, de la lipase en proportions telles que le granulé d'enzymes multiples obtenu présente une activité de lipase de 1 à 80 KLU/g, en particulier de 1,5 à 60 KLU/g, et/ou de la cellulase en proportions telles que le granulé d'enzymes multiples obtenu présente une activité de cellulase de 50 à 1250 CEVU/g, et en particulier, de 100 à 1000 CEVU/g.

6. Procédé selon une des revendications 1 à 5, caractérisé en ce que les matières porteuses sont sélectionnées parmi la cellulose, la maltodextrose, le saccharose, le sucre inverti, le glucose, les amidons, les farines de céréales, les éthers cellulosiques, l'aluminosilicate de métal alcalin, en particulier la zéolithe, le silicate stratifié, en particulier la bentonite ou la smectite, et les sels inorganiques ou organiques solubles dans l'eau, en particulier le chlorure, le sulfate, le carbonate ou l'acétate de métal alcalin.

7. Procédé selon une des revendications 1 à 6, caractérisé en ce que la matière porteuse contient de l'amidon en proportions de 20 à 80 % en poids, en particulier de 25 à 75 % en poids et de la farine de céréales, en quantités de 10 à 35 % en poids, en particulier de 15 à 20 % en poids, dans chaque cas par rapport à la totalité de la matière porteuse.

8. Procédé selon la revendication 7, caractérisé en ce que dans la matière porteuse, la somme des proportions de l'amidon et de la farine ne dépasse pas 95 % en poids, en étant en particulier comprise entre 60 et 95 % en poids.

9. Procédé selon une des revendications 1 à 8, caractérisé en ce que l'enzyme secondaire contient un sel inorganique, en particulier du sulfate et/ou du chlorure de métal alcalin, en proportions de 30 à 80 % en poids, de la cellulose en fibres ou en poudre, en quantités de 2 à 40 % en poids, ainsi que des liants, en particulier du dextrose, du saccharose, de l'alcool polyvinylique et/ou de la polyvinylpyrrolidone, en proportions de 0,1 à 15 % en poids.

10. Procédé selon une des revendications 1 à 9, caractérisé en ce que l'on introduit, après ou, en particulier, pendant le séchage, des matières pour l'enrobage et l'enduction des particules du produit d'extrusion, qui renferment éventuellement des colorants ou des pigments, en particulier du dioxyde de titane et/ou du carbonate de calcium.

11. Utilisation d'un granulé d'enzymes obtenable selon le procédé de l'une des revendications 1 à 10, pour la production de produits de lavage ou de nettoyage.
